# EUROPÄISCHE PATENTSCHRIFT

(11) **EP 0 579 970 B1**
(45) Veröffentlichungstag und Bekanntmachung des Hinweises auf die Patenterteilung: **08.03.2000**
(21) Anmeldenummer: 93110008.5
(22) Anmeldetag: 23.06.1993
(51) Int. Cl.: C07D 213/61

(54) **Verfahren zur Herstellung von 2-Chlor-5-aminomethylpyridin**
Process for the production of 2-chloro-5-aminomethylpyridine
Procédé pour la préparation de 2-chloro-5-aminométhylpyridine

(30) Priorität: 06.07.1992 DE 4222152
(43) Veröffentlichungstag der Anmeldung: 26.01.1994
(73) Patentinhaber: BAYER AG, 51368 Leverkusen (DE)
(72) Erfinder: Diehr, Hans-Joachim, D-42329 Wuppertall 11 (DE)

(56) Entgegenhaltungen:
- DE-A- 3 726 993
- DATABASE WPI Week 9236, Derwent Publications Ltd., London, GB; AN 92-299951
- DATABASE WPI Week 8651, Derwent Publications Ltd., London, GB; AN 86-336001
- DATABASE WPI Week 7913, Derwent Publications Ltd., London, GB; AN 79-24478B
- Book no. E7B, 1992, 'METHODEN DER ORGANISCHEN CHEMIE', HOUBEN-WEYL GEORG THIEME VERLAG, STUTTGART

## Beschreibung

Die Erfindung betrifft ein neues Verfahren zur Herstellung des bekannten 2-Chlor-5-aminomethyl-pyridins.

Es ist bekannt, daß man 2-Chlor-5-aminomethyl-pyridin, ein Zwischenprodukt für die Herstellung von Insektiziden erhält, wenn man 2-Chlor-5-cyano-pyridin mit Wasserstoff in Gegenwart von Raney-Nickel und von Ammoniak in einem Reaktionsmedium, welches Wasser und gegebenenfalls ein organisches Lösungsmittel enthält, bei Temperaturen zwischen 0°C und 60°C umsetzt (vgl. DE-OS 3726993).
Ausbeute und Qualität dieses Produktes sind beim angegebenen Verfahren jedoch nicht ganz zufriedenstellend.

Es wurde nun ein Verfahren zur Herstellung von 2-Chlor-5-aminomethyl-pyridin der Formel (I) 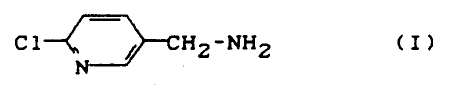 durch Umsetzung von 2-Chlor-5-cyano-pyridin der Formel (II) 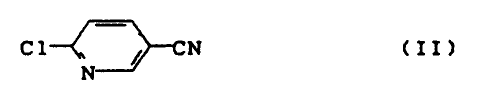 mit Wasserstoff in Gegenwart von Ammoniak und in Gegenwart eines wasserfreien Metallkatalysators, dadurch gekennzeichnet, daß die Umsetzung in einem unpolaren aprotischen Verdünnungsmittel bei Temperaturen zwischen 20°C und 150°C durchgeführt wird.

Es ist als überraschend anzusehen, daß die katalytische Hydrierung gemäß dem erfindungsgemäßen Verfahren in Gegenwart eines unpolaren aprotischen Verdünnungsmittels, d.h. ohne Verwendung von Wasser oder eines anderen protischen Lösungsmittels, das 2-Chlor-5-aminomethyl-pyridin in erheblich verbesserter Ausbeute, verglichen mit dem bekannten Verfahren, ergibt.

Der Reaktionsablauf beim erfindungsgemäßen Verfahren kann durch das folgende Formelschema wiedergegeben werden:

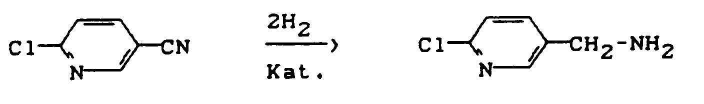

Das als Ausgangsstoff zu verwendende 2-Chlor-5-cyano-pyridin der Formel (II) ist bereits bekannt (vgl. J. Chem. Soc. [London] 1948, 1939-1945).

Das erfindungsgemäße Verfahren wird in Gegenwart eines Metallkatalysators durchgeführt. Vorzugsweise werden Raney-Katalysatoren, wie z.B. Raney-Nickel oder Raney-Cobalt eingesetzt. Raney-Cobalt wird als Metallkatalysator beim erfindungsgemäßen Verfahren besonders bevorzugt.

Das erfindungsgemäße Verfahren wird in Gegenwart eines unpolaren aprotischen Verdünnungsmittels durchgeführt. Hierunter sind in diesem Zusammenhang organische Lösungsmittel zu verstehen, welche keine leicht abspaltbaren Protonen enthalten. Hierzu gehören vorzugsweise aliphatische und aromatische, gegebenenfalls halogenierte Kohlenwasserstoffe, wie Hexan, Heptan, Octan sowie deren verzweigte Isomeren, ferner Cyclohexan, Methylcyclohexan, Benzol, Toluol, Xylol, Chlorbenzol und o-Dichlorbenzol. Besonders bevorzugt sind aromatische Kohlenwasserstoffe. Insbesondere genannt seien Toluol sowie Xylol und deren Gemische.

Die Reaktionstemperaturen können beim erfindungsgemäßen Verfahren in einem größeren Bereich variiert werden. Im allgemeinen arbeitet man zwischen 20°C und 150°C vorzugsweise zwischen 50°C und 120°C, insbesondere zwischen 80°C und 110°C.

Der Druck kann beim erfindungsgemäßen Verfahren ebenfalls in einem größeren Bereich variiert werden. Im allgemeinen arbeitet man im Druckbereich zwischen 1 und 200 bar, vorzugsweise zwischen 20 und 150 bar, insbesondere zwischen 50 und 120 bar.

Zur Durchführung des erfindungsgemäßen Verfahrens setzt man auf 1 Mol 2-Chlor-5-cyano-pyridin der Formel (II) im allgemeinen zwischen 20 ml und 200 ml, vorzugsweise zwischen 50 ml und 150 ml flüssigen Ammoniaks sowie zwischen 5 g und 50 g, vorzugsweise zwischen 10 g und 30 g eines Metallkatalysators ein.

Das erfindungsgemäße Verfahren kann nach üblichen Hydrierungsmethoden durchgeführt werden.

In einer bevorzugten Ausführungsform des erfindungsgemäßen Verfahrens werden das 2-Chlor-5-cyanopyridin, das inerte Verdünnungsmittel, der Metallkatalysator und das Ammoniak in einem Autoklaven vermischt und dann wird bei verschlossenem Autoklaven Wasserstoff unter Einstellen eines erhöhten Innendrucks zudosiert. Dann wird der Autoklaveninhalt bei erhöhtem Druck und erhöhter Temperatur bis zum Ende der Umsetzung gerührt.

Die Aufarbeitung kann auf übliche Weise durchgeführt werden. Vorzugsweise wird vom Katalysator abgesaugt und vom erhaltenen Filtrat das Lösungsmittel im Wasserstrahlvakuum sorgfältig abdestilliert.

Das zurückbleibende Rohprodukt kann durch Destillation unter vermindertem Druck gereinigt werden.

Das nach dem erfindungsgemäßen Verfahren herstellbare 2-Chlor-5-aminomethyl-pyridin der Formel (I) kann als Zwischenprodukt zur Herstellung von Hypotensiva (vgl. US-A 4499097) oder von Insektiziden (vgl. EP-A 425030) eingesetzt werden.

### Herstellungsbeispiele:

### Beispiel 1

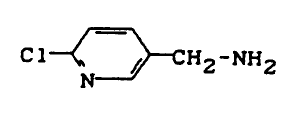

In einem Autoklaven werden 140 g (1,0 Mol) 2-Chlor-5-cyano-pyridin, 700 ml getrocknetes Toluol, 25 g getrocknetes Raney-Cobalt und 100 ml flüssigen Ammoniaks vermischt und in den verschlossenen Autoklaven wird dann bis zum Erreichen eines Innendrucks von 70 bar Wasserstoff eindosiert. Anschließend wird das Reaktionsgemisch im verschlossenen Autoklaven auf 100°C aufgeheizt und bei dieser Temperatur 8 Stunden gerührt (Innendruck: ca. 100 bar). Nach Öffnen des Autoklaven bei Raumtemperatur wird abgesaugt, der Filterkuchen (Katalysator) mit Toluol gewaschen und vom Filtrat das Verdünnungsmittel im Wasserstrahlvakuum sorgfältig abdestilliert.

Man erhält 130 g eines Rohproduktes, welches nach gaschromatographischer Analyse 91% 2-Chlor-5-aminomethyl-pyridin enthält.
Die Ausbeute beträgt damit 83% der Theorie.

### Beispiel 2

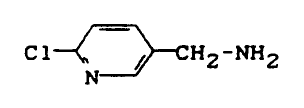

Beispiel 2 wird analog Beispiel 1 durchgeführt, wobei der Katalysator Raney-Cobalt durch die gleiche Gewichtsmenge Raney-Nickel ersetzt wird.

Ausbeute: 79% der Theorie.

## Patentansprüche (Patentansprüche für folgende(n) Vertragsstaat(en): AT, BE, CH, ES, FR, GB, IT, LI, NL)

1. Verfahren zur Herstellung von 2-Chlor-5-amino-methyl-pyridin der Formel (I) 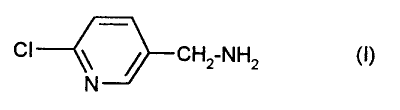 durch Umsetzung von 2-Chlor-5-cyano-pyridin der Formel (II) 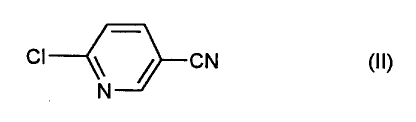 mit Wasserstoff in Gegenwart von flüssigem Ammoniak und in Gegenwart eines wasserfreien Metallkatalysators, dadurch gekennzeichnet, daß die Umsetzung in einem unpolaren aprotischen Verdünnungsmittel bei Temperaturen zwischen 20°C und 150°C durchgeführt wird.

2. Verfahren gemäß Anspruch 1, dadurch gekennzeichnet, daß als wasserfreie Metallkatalysatoren Raney-Nickel oder Raney-Cobalt eingesetzt werden.

3. Verfahren gemäß der Ansprüche 1 und 2, dadurch gekennzeichnet, daß als Verdünnungsmittel aliphatische und aromatische Kohlenwasserstoffe eingesetzt werden.

4. Verfahren gemäß der Ansprüche 1 und 2, dadurch gekennzeichnet, daß als Verdünnungsmittel Toluol oder Xylole eingesetzt werden.

## Patentansprüche (Patentansprüche für folgende(n) Vertragsstaat(en): DE)

1. Verfahren zur Herstellung von 2-Chlor-5-amino-methyl-pyridin der Formel (I) 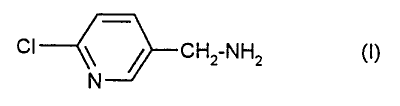 durch Umsetzung von 2-Chlor-5-cyano-pyridin der Formel (II) 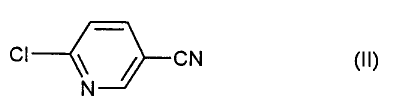 mit Wasserstoff in Gegenwart von flüssigem Ammoniak und in Gegenwart von wasserfreiem Raney-Cobalt, dadurch gekennzeichnet, daß die Umsetzung in einem unpolaren aprotischen Verdünnungsmittel bei Temperaturen zwischen 20°C und 150°C durchgeführt wird.

2. Verfahren gemäß Anspruch 1, dadurch gekennzeichnet, daß als Verdünnungsmittel aliphatische oder aromatische Kohlenwasserstoffe eingesetzt werden.

3. Verfahren gemäß Anspruch 1, dadurch gekennzeichnet, daß als Verdünnungsmittel Toluol oder Xylole eingesetzt werden.

## Claims (Claims for the following Contracting State(s): AT, BE, CH, ES, FR, GB, IT, LI, NL)

1. Process for the preparation of 2-chloro-5-amino-methyl-pyridine of the formula (I) 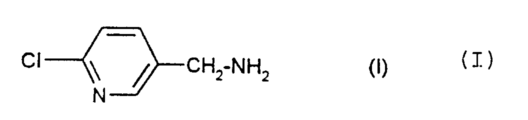 by a reaction of 2-chloro-5-cyano-pyridine of the formula (II) 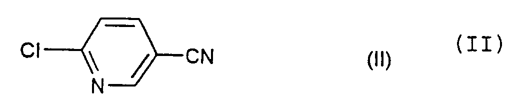 with hydrogen in the presence of liquid ammonia and in the presence of an anhydrous metal catalyst, characterised in that the reaction is carried out in an apolar aprotic diluent at temperatures between 20°C and 150°C.

2. Process according to Claim 1, characterized in that Raney nickel or Raney cobalt are used as the anhydrous metal catalysts.

3. Process according to Claims 1 and 2, characterised in that aliphatic and aromatic hydrocarbons are used as the diluent.

4. Process according to Claims 1 and 2, characterised in that toluene or xylenes are used as the diluent.

## Claims (Claims for the following Contracting State(s): DE)

1. Process for the preparation of 2-chloro-5-amino-methyl-pyridine of the formula (I) 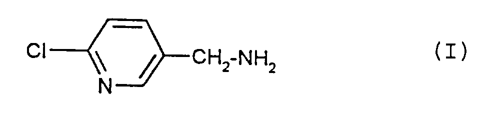 by a reaction of 2-chloro-5-cyano-pyridine of the formula (II) 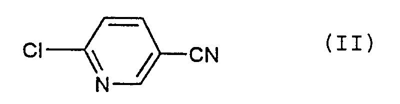 with hydrogen in the presence of liquid ammonia and in the presence of anhydrous Raney cobalt, characterised in that the reaction is carried out in an apolar aprotic diluent at temperatures between 20°C and 150°C.

2. Process according to Claim 1, characterized in that aliphatic or aromatic hydrocarbons are used as the diluent.

3. Process according to Claims 1, characterised in that toluene or xylenes are used as the diluent.

## Revendications (Revendications pour l'(les) Etat(s) contractant(s) suivant(s): AT, BE, CH, ES, FR, GB, IT, LI, NL)

1. Procédé de production de 2-chloro-5-amino-méthylpyridine de formule (I) 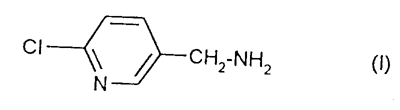 par réaction de la 2-chloro-5-cyanopyridine de formule (II) 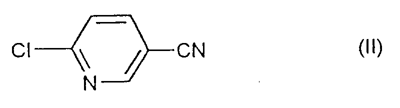 avec l'hydrogène en présence d'ammoniac liquide et en présence d'un catalyseur métallique anhydre, caractérisé en ce que la réaction est conduite dans un diluant aprotique non polaire à des températures comprises entre 20°C et 150°C.

2. Procédé suivant la revendication 1, caractérisé en ce que du nickel de Raney ou du cobalt de Raney sont utilisés comme catalyseurs métalliques anhydres.

3. Procédé suivant les revendications 1 et 2, caractérisé en ce que des hydrocarbures aliphatiques et aromatiques sont utilisés comme diluants.

4. Procédé suivant les revendications 1 et 2, caractérisé en ce que du toluène ou des xylènes sont utilisés comme diluants.

## Revendications (Revendications pour l'(les) Etat(s) contractant(s) suivant(s): DE)

1. Procédé de production de 2-chloro-5-amino-méthylpyridine de formule (I) 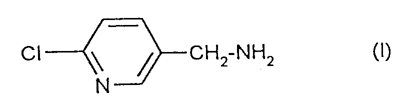 par réaction de la 2-chloro-5-cyanopyridine de formule (II) 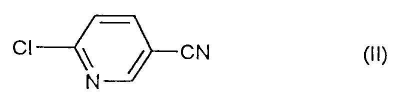 avec l'hydrogène en présence d'ammoniac liquide et en présence de cobalt de Raney anhydre, caractérisé en ce que la réaction est conduite dans un diluant aprotique non polaire à des températures comprises entre 20°C et 150°C.

2. Procédé suivant la revendication 1, caractérisé en ce que des hydrocarbures aliphatiques ou aromatiques sont utilisés comme diluants.

3. Procédé suivant la revendication 1, caractérisé en ce que du toluène ou des xylènes sont utilisés comme diluants.
